# EUROPEAN PATENT SPECIFICATION

(11) **EP 1 234 543 B1**
(45) Date of publication and mention of the grant of the patent: **20.04.2011**
(21) Application number: 01954415.4
(22) Date of filing: 02.08.2001
(51) Int. Cl.: A61B 10/00

(54) **SAMPLER**
PROBENENTNEHMER
ECHANTILLONNEUR

(30) Priority: 04.08.2000 JP 2000237176; 13.03.2001 JP 2001070956
(43) Date of publication of application: 28.08.2002
(73) Proprietor: Olympus Optical Co., Ltd., Tokyo 151-0072 (JP)
(72) Inventor: ISHIZAKA, Akitoshi, Shinagawa-ku, Tokyo 141-0022 (JP); SAITO, Tatsuya, Setagaya-ku, Tokyo 156-0055 (JP)
(74) Representative: Hübner, Helmut
(86) International application number: PCT/JP2001/006669
(87) International publication number: WO 2002/011619

(56) References cited:
- EP-A- 0 612 503
- WO-A-00/04833
- FR-E- 58 319
- JP-A- 1 131 641
- JP-A- 2 074 243
- JP-A- 2 224 651
- JP-A- 59 082 835
- JP-U- 54 013 292
- US-A- 5 031 635
- US-A- 5 722 423
- US-A- 5 738 109
- US-A- 5 922 614

## Description

### Background Art

### (1) Field of the Invention

The present invention relates to a sampling tool for taking a sample such as mucus from a living body and a method of transferring a substance into a living body.

### (2) Prior Art

When a mucus sample is taken from a body, a probe has been used so far. An absorber employed in the probe for absorbing the mucus has a predetermined size and shape. Therefore, it has been actually difficult to alter the size of the absorber into a desired size. This is because materials different in size have to be prepared for each absorber.

Japanese Patent Application No. 2001-137248 discloses a sampling tool for taking exudates from the bronchus. However, such a sampling tool is used in couple with an endoscope and under the endoscope. In such a sampling tool, a sampling section is usually inserted into a peripheral part of the bronchus and allowed to take the mucus. The sampling section is generally formed of a cotton scrub, which is prepared by winding cotton around a paper shaft, and a foaming material with a supporting shaft. When the sampling section is pushed out from the tip portion of a catheter and brought into contact with a target site, it can absorb the mucus or the like.

Japanese Patent Application No. 2001-137248 discloses a sampling tool in which an absorber is attached to the tip of a long member slidably moving through an outer sheath. In this case, the size and shape of the absorber is initially defined. Therefore, it is necessary to select an outer sheath having an inner diameter large enough to fit the absorber therein.

However, since a sampling tool is loaded in an endoscope, the outer diameter of the outer sheath is limited by the diameter of the forceps insertion channel of an endoscope. Accordingly, the inner diameter of the outer sheath is also limited. As a result, an absorber capable of absorbing a desired amount of liquid cannot be used in some practical cases.

On the other hand, another tool, which is not used in a body, like the sampling tool mentioned above, is disclosed in Japanese Patent Application No. 7-194617. This is a cleaning tool having a brush and a sponge for cleaning the forceps insertion channel of an endoscope and has been already used widely. Also in the cleaning tool, the diameter of a sponge is initially determined by the sizes of parts. It is therefore difficult to use such a cleaning tool in the forceps insertion channel of an endoscope if the size of the channel differs from the cleaning tool.

A conventional sampling tool uses a sampling section formed of cotton and a foaming material to absorb a sample such as mucus. Therefore, the amount of the sample changes in proportional to the volume of the sampling section and limited by the size of a paper shaft and a supporting shaft to which the sampling section (cotton or foaming material) is to be attached. More specifically, the amount of a sample is inevitably reduced by the volume corresponding to a rod or a support shaft used in the sampling section.
A conventional sampling tool has a problem in that the sampling amount is low. This is a significant demerit in especially obtaining biological data.

However, it is extremely difficult to manufacture a sampling section as thin as possible while maintaining a sampling amount, in a technical point of view.

Furthermore, since the sampling section is formed of cotton or a foaming material, a sample is taken at an extremely low speed. This means that the sampling section stays in contact with a target site within a body (especially in lung) for a long time, applying a load on a patient.

Document EP 0 612 503 relates to a universally applicable cell collecting device with a handle and receiving means with substantially parallel bristles which are arranged with a considerably wide gap between each other.

Document US 5 738 109 describes a device for collecting cell samples from internal organs. A catheter comprises a brush the bristles of which are arranged around the circumference and can be housed in a retractable sleeve.

In document FR 58 319 a bundle of resilient fiber filaments is provided with its end portion in an attaching portion of a metallic rod.

The present invention has been made in view of the aforementioned problems. An object of the present invention is to provide a sampling tool capable of obtaining a large amount of specimen in a short time.

### Disclosure of Invention

This object is solved by the subject matter of claim 1.

The sampling tool of the present invention has a shaft member having a distal end and a proximal end and an absorber arranged in a tip portion of the shaft material as defined in claim 1. The absorber is a bundle of a plurality of fiber filaments. The sampling tool may have an outer sheath for loading and unloading the shaft member.

### Brief Description of Drawings

FIG. 1A is a perspective view showing an entire sampling tool according to a first embodiment of the background of the present invention;
FIG. 1B is a longitudinal sectional view of the sampling tool according to the first embodiment of the background of the present invention.
FIG. 2 is a perspective view of a sampling unit of the sampling tool according to the first embodiment of the background of the present invention.
FIGS. 3A and 3B are perspective views of a distal unit of the sampling tool according to the first embodiment of the background , showing an absorber sandwiched by a wire body;
FIG. 4 is a side view of the distal unit of the sampling tool according to the first embodiment of the background , showing an absorber sandwiched by a wire body;
FIG. 5 is a side view of the distal unit of the sampling tool according to the first embodiment of the background , showing the process of fastening an absorber by the wire body;
FIG. 6 is a side view of the distal unit of the sampling tool according to the first embodiment of the background , showing an absorber fitted to the wire body;
FIG. 7 is a perspective view of a wire body of the distal unit of the sampling tool according to the first embodiment of the background ;
FIG. 8 is a perspective view of an absorber to be fitted to the wire body of the distal unit of a sampling tool according to the second embodiment of the background ;
FIG. 9 is a side view of an absorber fitted to the wire body of the distal unit of the sampling tool according to the second embodiment of the background
FIG. 10 is a perspective view of a sampling tool to be fitted to the wire body of the distal unit of a sampling tool according to a third embodiment of the Background;
FIG. 11 is a side view of the distal unit of the sampling tool according to the third embodiment of the background , showing the state of an absorber fitted to the wire body;
FIG. 12 is a perspective view of a sampling tool according to a fourth embodiment which forms part of the present invention;
FIG. 13 is a longitudinal sectional view of a sampling tool according to a which forms part fourth embodiment of the present invention;
FIG. 14 is a perspective view of an absorber of the sampling tool according to a fourth embodiments which forms part of the present invention;
FIG. 15 is a perspective view of the absorber of the sampling tool according to the fourth embodiment which forms part of the present invention;
FIG. 16 is an explanatory view of the sampling tool according to the fourth embodiment which forms part of the present invention, showing the operation thereof;
FIG. 17 is a perspective view of the absorber of the sampling unit of a sampling tool according to a fifth embodiment which forms part of the present invention;
FIG. 18 is a perspective view of the absorber of the sampling unit of the sampling tool according to a sixth embodiment which forms part of the present invention;
FIG. 19 is a perspective view of the absorber of the sampling unit of a sampling tool according to a seventh embodiment which forms part of the present invention;
FIG. 20 is a longitudinal sectional view of the sampling unit of a sampling tool according to an eighth embodiment not forming part of the present invention of the present invention;
FIG. 21 is a longitudinal sectional view of the sampling unit of a sampling tool according to a ninth embodiment not forming part of the present invention of the present invention;
FIG. 22 is a longitudinal sectional view of the sampling unit of a sampling tool according to a tenth embodiment not forming part of the present invention of the present invention;
FIG. 23 is a perspective view of the absorber of the sampling unit of a sampling tool according to a tenth embodiment not forming part of the present invention of the present invention;
FIG. 24 is a longitudinal sectional view of the absorber of the sampling unit of a sampling tool according to an eleventh embodiment which forms part of the present invention; and
FIG. 25 is a perspective view of the absorber of the sampling unit of a sampling tool according to an eleventh embodiment which forms part of the present invention

### Best Mode for Carrying Out of the Invention

### [First embodiment of the background of the invention

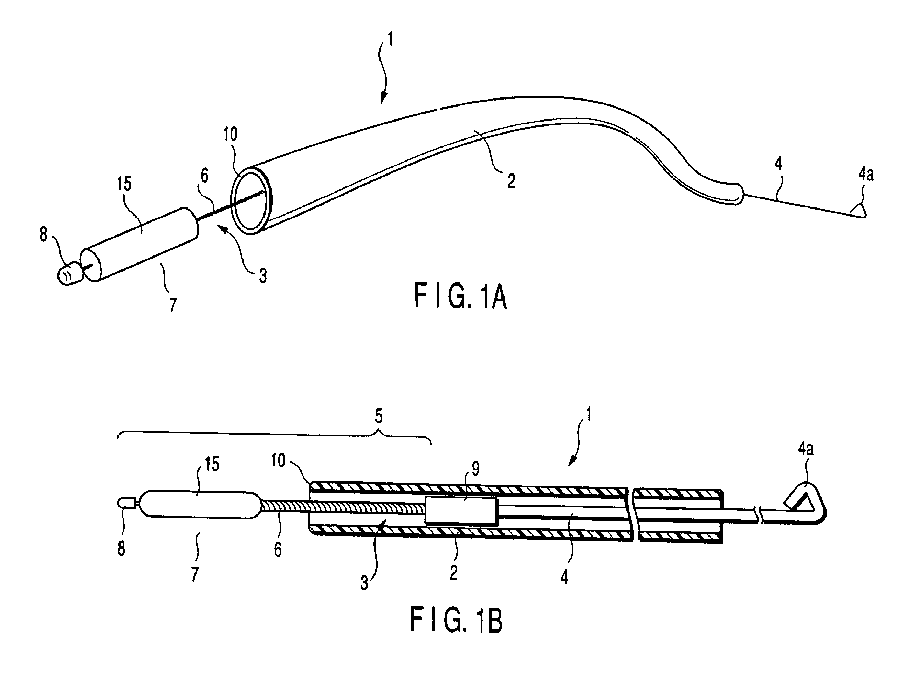

A sampling tool according to the first embodiment of the background will be explained with reference to FIGS. 1A to 7.

### (Structure)

FIGS. 1A and 1B shows the entire structure of a sampling tool 1. The sampling tool 1 has a long flexible tube, an outer sheath 2 and a sampling unit 3 to be inserted into the outer sheath 2.

The sampling unit 3 has an operating wire 4 and a distal unit 5. The distal unit 5 is formed of a sampling portion 7 fitted at the middle portion of a long wire body 6. A tip 8 is formed of an X-ray impermeable material and mechanically fitted to the front-end of the wire body 6. The wire body 6 is a component of a shaft member. Conversely, the shaft member may have the wire body 6 as a part. The shaft member is movable along the outer sheath 2, lengthwise.

As shown in FIG. 1B, the proximal end of the wire body 6 of the distal unit 5 is connected to the tip of the operation wire 4 by means of a joint 9. The joint 9 is a tubular member formed over the proximal end of the wire body 6 and the tip of the operation wire 4 and tightly connects them by cramping or with a solder and an adhesive agent.

The front end 10 of the outer sheath 2 is rounded. A handle portion 4a is formed by folding the proximal portion of the operation wire 4 into a loop.

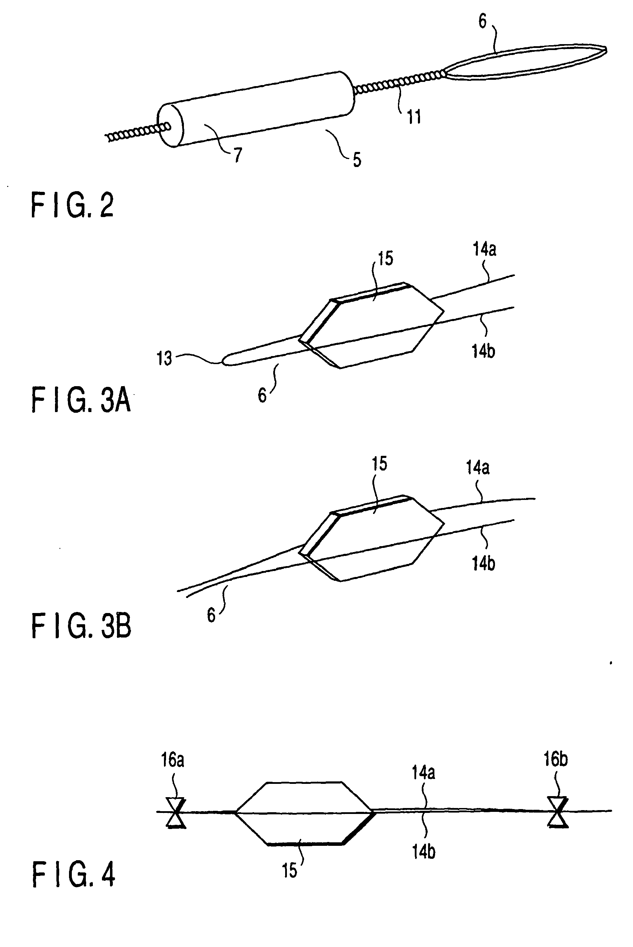

The structure of the distal unit 5 will be described more specifically below. FIG. 2 shows the wire body 6 of the distal unit 5. A shaft 11 is formed of elemental wires helically laid up together. The sampling section 7 is attached around the shaft 11.

In this case, an absorber 15 serving as a sampling tool 1 is sandwiched between two wires 14a and 14b formed by folding a single wire in the mid point (to folding point 13) into two as shown in FIG. 3A. Alternatively, the absorber 15 may be sandwiched by a plurality of discrete wires (two wires are shown) 14a and 14b, as shown in FIG. 3B. The absorber 15 is formed of a continuous foaming body such as polyurethane.

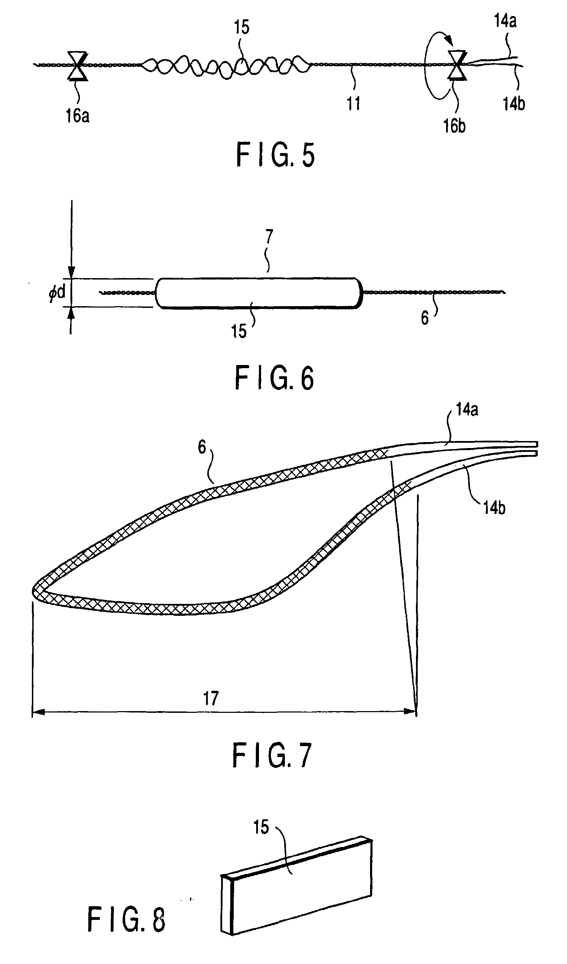

Furthermore, as shown in FIG. 4, the wires 14a and 14b are clamped at both sides of the absorber 15, at points which are separated from the absorber by a predetermined distance. If the rear-side clamp position 16b is twisted and rotated while a foreside clamp position 16a is fixed as shown in FIG. 5, the wire body 6 is twisted around the shaft and wires 14a and 14b are intertwined with each other to sandwich the absorber 15. In this way, the absorber is gradually fastened.

The expandable, elastic and restorative absorber 15 is squeezed and distorted, forming a sampling section 7 into an almost cylindrical form, as shown in FIG. 6.

Since the hexagonal absorber 15 is used as shown in FIGS. 3A and 3B, the resultant shape of the absorber 15 becomes substantially a cylindrical form. The front end and the rear end of the absorber 15 are formed vertically spherically.

A tip 8 formed of an X-ray impermeable material is provided at forefront portion thereof. The outer surface of the tip 8 is spherically formed.

If the absorber is formed while rotating the rear-end clamp position 16b at a different speed, the outer diameter d of the sampling section 7 changes. In this way, the outer diameter d of the sampling section 7 can be set at a desired size (See FIG. 6). In other words, if the number of twisting the wires 14a and 14b is changed, the diameter of the absorber 15 can be changed, thereby controlling the size of the outer diameter within the range of 0.5 mm to 3 mm.

If the porosity of the absorber 15 is set at 40% or more, the sampling tool capable of taking a sample more efficiently.

The absorber is desirably set so as to absorb a medium in an amount of 5 micro liters to 60 or more micro liters in terms of water.

As shown in FIG. 7, the wire body 6 has a flexible portion 17 (longer than the lengthwise size of the absorber 15), which is softly processed by annealing. Due to the presence of the flexible portion 17, the wires 14a and 14b are more easily entangled with each other when the wires 14a and 14b are rotated.

### (Function)

How to introduce the sampling tool 1 (shown in FIGS. 1A and 1B) into a body through an endoscope will be explained. In the first place, the sampling unit 3 is housed in the outer sheath 2. Subsequently, the tip of the outer sheath 2 of the sampling tool is made to be approached a target site which has been captured by the endoscope previously inserted into the body cavity. As shown in FIG. 1B, the operator grabs the handling portion 4a of the operation wire 4 while the outer sheath 2 is held, and pushes the operation wire 4 forward. In this manner, the sampling section 7 of the sampling unit 3 protrudes from an opening of the distal end of the outer sheath 2. Subsequently, the operator further pushes the sampling section 7 against the target site to take a sample. Thereafter, a sample unit 3 is loaded into the outer sheath 2 and then the sampling tool 1 is removed from the endoscope.

The aforementioned method can be used for sampling the respiratory epithelial mucus of the lung. If the respiratory epithelial mucus taken as a sample by the sampling tool 1 is analyzed for protein mediators such as cytokine and arachidonic acid metabolite, lipid mediators such as a platelet-activating factor, it is possible to biologically diagnose a disease. On the other hand, the respiratory epithelial cell taken as a sample can be cultured.

Note that, after the sample unit 3 is removed from an endoscope, the sampling section 7 and the absorber 15 are separated from the shaft member. The sampling section 7 is further subjected to the following treatment.

### (Effect)

According to the embodiment, since the outer diameter of the sampling section 7 can be changed, the size of the absorber 15 may be changed and the inner diameter of the outer sheath 2 for housing the sampling portion 7 can be arbitrarily changed. If the size of the absorber 15 varies, the inner diameter of the outer sheath 2 has to be changed in accordance with the size of the absorber 15 in a conventional case, however, in this embodiment, the absorber needs not to be prepared depending upon the size. In addition, the device can be reduced in diameter. Furthermore, if the degree of twisting a wire is changed when the absorber 15 is formed, the amount of liquid absorbed by the absorber can be varied.

### [Second Embodimentof the background of the invention

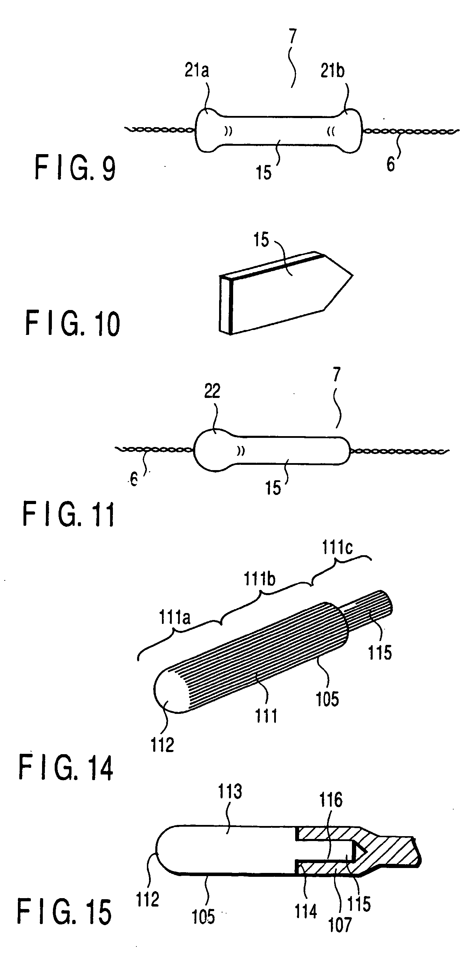

With reference to FIGS. 8 and 9, a sampling tool according to a second embodiment of the background will be explained.

### (Structure)

A basic structure is the same as that of the first embodiment described below. The original shape of the absorber 15 is a rectangular parallelepiped, as shown in FIG. 8. When the wire body 6 is twisted to form the absorber 15, the resultant shape of the absorber, that is, the sampling section 7, becomes a cylinder with spherical bumps 21a and 21b at the forefront end and the backend, due to the rectangular parallelepiped, as shown in FIG. 9.

### (Function and Effect)

The same function and effect as those of the first embodiment can be obtained. Since bumps 21a and 21b are formed at the forefront end and backend of the sampling section 7, respectively, a sample can be efficiently taken by gently operating the sampling section even if the sample is present in a small amount. As a result, the absorption amount of liquid can be increased. Furthermore, if a sampling target site is a tubular cavity, the sampling tool can remain at the cavity.

### [Third Embodiment of the background of the invention

A sampling tool according to a third embodiment of of the background will be explained with reference to FIGS. 10 and 11.

### (Structure)

The basic structure of the sampling tool is the same as that of the first embodiment or the second embodiment except for the shape of the absorber 15. The absorber 15 is formed into a home base shape as shown in FIG. 10. In this case, when the wire body 6 is twisted to form the absorber 15, a spherical bump 22 is formed only at the forefront of the absorber, as shown in FIG. 11. The backend of the absorber has substantially a cylindrical form, as is the same as in the first embodiment.

### (Function and Effect)

The same function and effect as those of the first or second embodiment can be obtained. Sampling can be efficiently made by the tip of the absorber 15. In addition, the absorber can be in multi contact with a target site due to the presence of the bump at the tip.

### [Fourth embodiment]

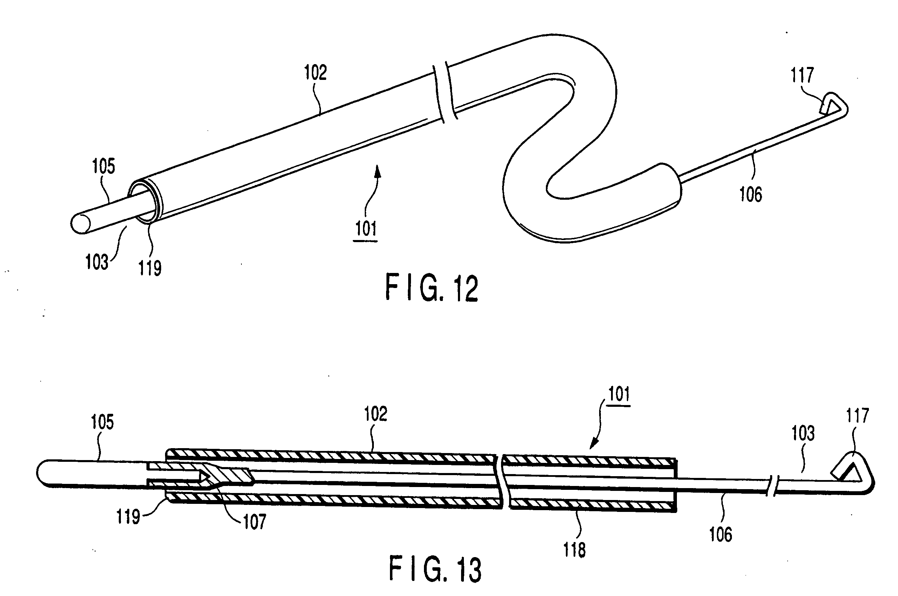

A sampling tool according to a fourth embodiment which forms part of the present invention will be described with reference to FIGS. 12-15.

### (Structure)

As shown in FIG. 12, a sampling tool 101 is formed by slidably inserting a sampling unit 103 into a long flexible outer sheath 102.

The sampling unit 103 is formed as shown in FIG. 13. An absorber 105 serving as a sampling tool is positioned at the forefront side. A longitudinal operation wire 106 formed of an X-ray impermeable material is positioned at the proximal side. They are tightly connected by means of a joint 107 formed of the same X-ray impermeable material as used in the operation wire by cramping or with a solder or an adhesive agent.

The absorber 105 is formed of a bundle of chemical fiber filaments of polyester or the like, having outer diameters of 0.1 mm or less as shown in FIG. 14.

The absorber 105 is formed of a front-end portion 111a, a middle portion 111b, and a rear-end portion 111c. According to the invention, the absorber 105 is formed by adhering a plurality of fiber filaments in part. Each of the fiber filaments is resilient, the fiber filaments may be used as they are.

The tip portion of the front-end portion 111a is rounded because the front-end portion 111a is brought into contact with a living body. The middle portion 111b is equivalent to a cylindrical portion 113. Since the rear-end portion 111c is connected to the joint 107, a small-diameter portion 115 having a flange end 114 is formed. The small-diameter portion 115 is inserted into a recess portion (hole) 116 of the joint 107 as shown in FIG. 15 and mechanically fixed.

The rear-end portion of the operation wire 106 protrudes from the outer sheath 102. The proximal portion is folded to form a folded portion 117 to protect the operator from being injured.

The outer sheath 102 is formed of a material 118 usually used in a tube. The distal end 119 of the material 118 is rounded.

### (Function)

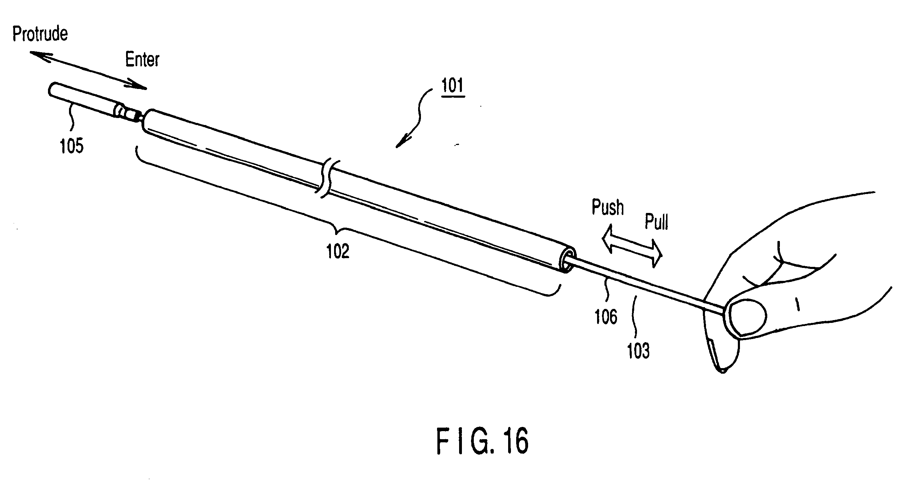

How to operate a sampling tool 101 according to this embodiment will be explained. As shown in FIG. 16, a sampling tool 101 is introduced into the body cavity through an endoscope. Thereafter the operation wire 106 of the sampling unit 103 is pressed inward to push out the absorber 105 from the front-end of the outer sheath 102. In this manner, the absorber 105 is brought into contact with a target site of the absorber 105. Thereafter, the mucus is absorbed by the absorber 105.

Since the absorber 105 is formed of a bundle of the chemical fiber filaments 111 having outer diameters of 0.1 mm or less, a liquid such as mucus is sucked up through spaces between fiber filaments by use of the capillary action.

After sampling is completed, the operation wire 106 is pulled to load the absorber 105 into the outer sheath 102. Subsequently, the sampling tool 101 housing the absorber 105 therein is removed from the endoscope.

Thereafter, the absorber 105 of the sampling tool 101 is dipped in a test solution to elute the sample thus taken and disperse it the test solution. Since the absorber 105 is formed of a chemical fiber bundle 111, the mucus is taken as a sample at a high speed, and quickly eluted and dispersed in the detection solution.

The aforementioned method may be used when the respiratory epithelial mucus is taken as a sample from the lung as is the case of the first embodiment. In this case, if the respiratory epithelial mucus thus taken by the sampling tool 101 is analyzed for a protein mediator such as cytokine or an arachidonic acid metabolite, a lipid mediator such as a platelet-activating factor, a disease can be biologically diagnosed. In addition, the respiratory endothelial mucus cells can be cultured.

### (Effect)

The absorbing material forming the absorber 105 is formed by bundling micro fiber filaments. The mucus is sucked up due to the capillary action of pores or slits between fiber filaments. Therefore, a sufficient amount of a sample such as mucus can be obtained. If the length and diameter of the absorber 105 are changed, the amount of the sample can be easily increased or decreased.

By virtue of the capillary action, the mucus can be sucked up at a high speed. Furthermore, the sampling tool can come into contact with a target site in a short time during the sampling.

### [Fifth embodiment]

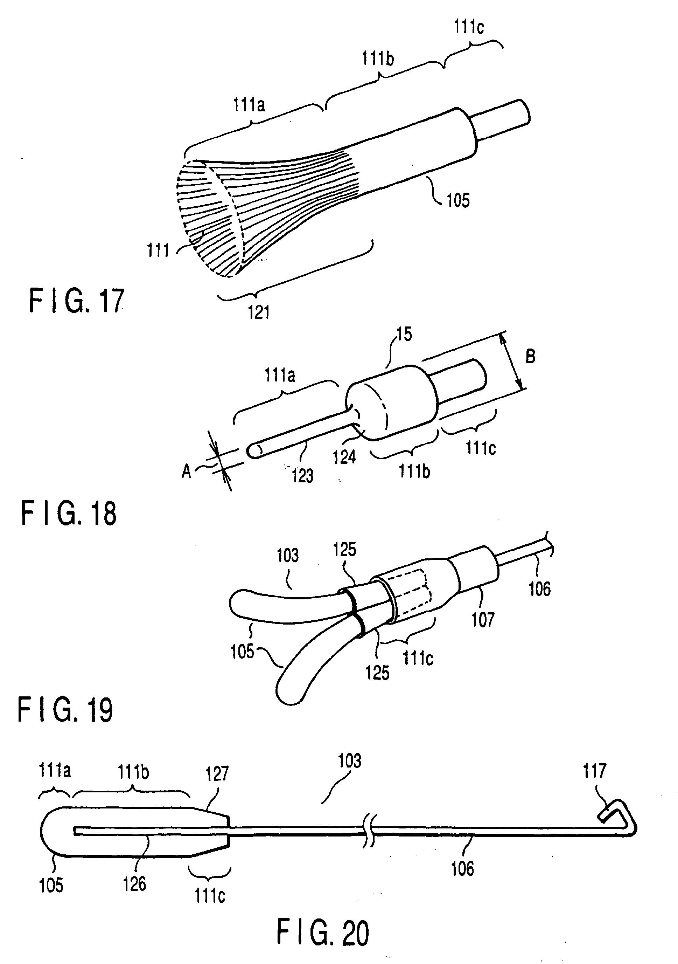

A sampling tool according to the fifth embodiment which forms part of the present invention will be explained with reference to FIG. 17.

### (Structure)

The basic structure of this embodiment is the same as in the fourth embodiment. The structure of an absorber 105 herein is modified as shown in FIG. 17.

The absorber 105 of this embodiment has a brush form front-end portion 111a. More specifically, the chemical fiber filaments 111 are loosen or disentangled to form a brush portion 121. In this respect, the absorber 105 of this embodiment differs from that of the fourth embodiment. Other structures including the middle portion 111b and the rear-end portion 111c are the same as those of the fourth embodiment shown in FIG. 14.

### (Function)

The function is the same as described in the fourth embodiment.

### (Effect)

A sample can be taken from a broad range around a lesion.

### [Sixth embodiment]

A sampling tool according to the sixth embodiment will be explained with reference to FIG. 18.

### (Structure)

The basic structure of this embodiment is the same as in the fourth embodiment or the fifth embodiment. The structure of an absorber 105 herein is modified as shown in FIG. 18.

In the absorber 105, a front-end small-diameter portion 123b, that is, the outer diameter (A) of a front-end portion 111a is smaller than the outer diameter (B) of the middle portion 111b. In this respect, this embodiment differs from previous embodiments. The middle portion 111b and the rear end portion 111c are the same as those in the fourth embodiment.

The stepped portion 124 between the front-end small-diameter portion 123 and the middle portion 111b is quite smoothly rounded.

### (Function)

The function is the same as described in the fourth embodiment.

### (Effect)

Since the front-end small-diameter portion 123 is formed, pinpoint sampling can be performed. When the sampling tool is used in the bronchus, sampling at a narrow cavity is successfully performed.

### [Seventh embodiment]

A sampling tool according to the seventh embodiment of the present invention will be explained with reference to FIG. 19.

### (Structure)

The basic structure of this embodiment is the same as in the fourth embodiment or the fifth embodiment. The structure of an absorber unit 103 herein has at least two absorbers 105, which are extended outward from the operation wire, as shown in FIG. 19. In this respect, the structure of the seventh embodiment differs from those of the fourth to sixth embodiments.

The structure of each absorber is the same as that shown in the fourth embodiment. The rear-end portion 111c is covered with a covering member 125 and mechanically connected tight to the joint 107.

### (Function)

The function is the same as described in the fourth embodiment.

### (Effect)

According to this embodiment, the sampling can be made in a body cavity by making a single-approach to a desired lesion.

### [Eighth embodiment]

The sampling tool according to the eighth embodiment not forming part of the present invention will be described with reference to FIG. 20.

### (Structure)

The structure of a sampling unit 103 of this embodiment differs from that of any one of the fourth embodiment to seventh embodiment. As shown in FIG. 20, an operation wire 106 is inserted to the core of the absorber 105. The tip portion of the operation wire 106 is inserted deep up to the fore-end portion of the absorber 105 to form a connecting portion 126.

The connecting portion 126 is connected tight to the absorber 105 by adhesion or heat welding. Alternatively, after the tip portion of the operation wire 106 is inserted into the absorber 105, the absorber may be formed.

The front-end portion 111a and the middle portion 111b of the absorber, 105 have the same structures as those shown in the embodiment 4. The rear-end portion 111c has a tapered portion 127 whose outer diameter is reduced rearward.

### (Function)

The basic function is the same as in the fourth embodiment. This embodiment has the following intrinsic function. Since the operation wire 106 is formed of an X-ray impermeable material, it can be accurately made to approach to the shade of a lesion, when sampling is performed under the X-ray observation.

When a sampling unit 103 is smoothly loaded into an outer sheath 102 due to the presence of the taper portion 127 formed at the rear-end portion 111a of the absorber 105, after the sampling.

### (Effect)

The tip portion of the absorber 105 is identified under X-ray perspective observation. The absorber 105 is connected to the operation wire 106 without using a connection means. Therefore, the number of parts can be decreased.

### [Ninth embodiment]

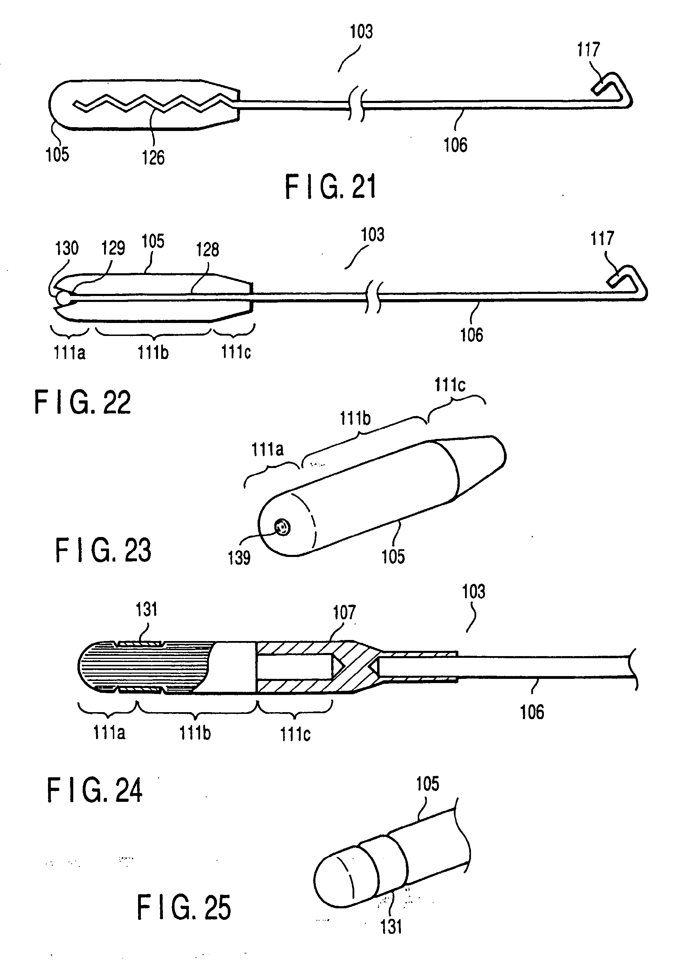

The sampling tool according to the ninth embodiment not forming part of the present invention will be described with reference to FIG. 21.

### (Structure)

The structure of this embodiment is the same as the embodiment 8 except that a connection portion 126 is formed of the forefront portion of an operation wire 106 which is spirally formed and inserted in the absorber 105, as shown in FIG. 21.

The connecting portion 126 of the operation wire 106 is inserted into substantially the core of the absorber 105 while rotating it, thereby connecting them. Other structures and the connection method are the same as those in the eighth embodiment.

### (Function)

The function is the same as in the eighth embodiment.

### (Effect)

The effect of the present invention is the same as that of the eighth embodiment. In addition, the absorber 105 is connected to the operation wire 106 with reliability. There is a low possibility that the absorber comes off.

### [Tenth embodiment]

The sampling tool according to the tenth embodiment not forming part of the present invention will be explained with reference to FIGS. 22 and 23.

### (Structure)

The basic structure of this embodiment is the same as that of the eighth or ninth embodiments and differs in the following points.

As shown in FIGS. 22 and 23, a through-hole 128 is formed in the core portion and extended along the shaft from the rear-end portion 111c to the front-end portion 111a. The center of the tip of the absorber 105 has a tip taper 129 whose outer diameter is gradually reduced toward the rear unit. The tip taper 129 communicates with the through-hole 128.

The tip portion of the operation wire 106 is inserted into the through hole 128. At the tip portion of the operation wire 106, a tip spherical portion 130 is formed which is larger than the through-hole 128 in diameter. The tip spherical portion 130 is held by the tip-taper 129 of the absorber 105. The tip spherical portion 130 is formed by fixing a discrete-form spherical member to the operation wire 106 or by melting the tip of the operation wire 106 with plasma. The absorber 105 and the operation wire 106 are connected tight with an adhesive agent or heat welding.

### (Function)

The function is the same as in the eighth and ninth embodiments.

### (Effect)

The same effects as in the eighth and ninth embodiments can be obtained. In addition, since an X-ray impermeable maker is positioned at the tip portion of the absorber 105, a sampling tool is more accurately guided to a target site.

### [Eleventh embodiment]

The sampling tool according to the eleventh embodiment of the present invention will be described with reference to FIGS. 24 and 25.

### (Structure)

The basic structure of the embodiment is the same as that described in any one of the fourth to sixth embodiments except that an X-ray impermeable member 131 is added to an absorber 105.

As shown in FIGS. 24 and 25, a ring-form X-ray impermeable member 131 formed of stainless or platinum is attached to a portion between a distal-end portion 111a and a middle portion 111b of an absorber 105 so as to cover the absorber 105.

The X-ray impermeable member 131 is fixed to the absorber 105 by cramping or with an adhesive agent or engaged with the absorber 105.

### (Function)

The function of this embodiment is the same as that described in any one of the eighth to the tenth embodiments.

### (Effect)

The effect of this embodiment is the same as that of the tenth embodiment. In addition, the position of the X-ray impermeable marker can be arbitrarily changed.

Note that the present invention is not limited to the embodiments and applicable to other embodiments.

For example, a substance may be transferred to a target lesion within the body. In this case, an absorber impregnated with a substance such as a medicament or a physiologically active substance, or an aqueous solution containing a gene, is loaded in an outer sheath. The sampling tool thus prepared is introduced into the body cavity through an endoscope. When the endoscope reaches a target lesion, an absorber is pushed out from the outer sheath. After a substance contained in the absorber is transferred to the lesion, the absorber is loaded into the outer sheath and removed together with the endoscope.

The present application incorporate contents of Japanese Patent Application No. 2000-237176 filed April 4, 2000 and Japanese Patent Application No. 2001-70956 filed March 13, 2001.

## Claims

1. A sampling tool (101) comprising:
a shaft member (106) having a distal end and a proximal end;
an absorber (105) arranged at least at the distal end of the shaft member (106) and connected to the shaft member (106),
said absorber (105) being a bundle of a plurality of resilient fiber filaments(111) extending in a longitudinal direction from the distal end of the shaft member formed by adhering the plurality of fiber filaments (111) in part so that the mucus is able to be sucked up due to the capillary action of pores and/or slits between the fiber filaments, and having a front-end portion (111 a) for contacting a target site, and
an outer sheath (102) into which said absorber (105) is movable along a shaft direction of the shaft member so that the absorber is able to be pushed out from the front-end of the outer sheath (102) for bringing the absorber (105) in contact with the target site.

2. A sampling tool (101) according to claim 1 wherein said absorber (105) has the front-end portion (111a) with a rounded tip portion for contacting the mucus to be absorbed.

3. A sampling tool(101) according to claim 1 wherein said absorber has a middle portion (111b) which is equivalent to a cylindrical portion and a rear-end portion (111c) connected to the shaft member (106).

4. A sampling tool (101) according to any one of the preceding claims wherein said fiber filaments have outer diameters of 0.1 mm or less.

5. A sampling tool (101) according to any one of the preceding claims wherein said absorber (105) has a porosity of 40% or more.

6. A sampling tool(101) according to any one of the preceding claims wherein said absorber (105) has an outer diameter of 0.5 mm to 3 mm.

7. A sampling tool(101) according to any one of the preceding claims, wherein said shaft member further comprises an X-ray impermeable member (131) arranged to the distal end of said shaft member:

8. A sampling tool (101) according to claim 1, which further comprises:
guiding means guiding the absorber (105) into a body cavity through an endoscope; and
pushing means pushing the absorber (105) out of the outer sheath (102) at a target lesion.

9. A sampling tool(101) according to any one of the preceding claims wherein said absorber (105) can absorb 5 to 60 or more micro liters of medium in terms of water.

10. A sampling tool (101) according to any one of the preceding claims wherein the shaft member (106) is a wire the proximal portion of which is folded to form a folded portion (117).

## Patentansprüche

1. Ein Probenahme-Werkzeug (101), das aufweist:
ein Schaftelement (106) mit einem distalen Ende und einem proximalen Ende,
eine Absorptionseinrichtung (105), die zumindest an dem distalen Ende des Schaftelements (106) angeordnet und mit dem Schaftelement (106) verbunden ist,
wobei die Absorptionseinrichtung (105) ein Bündel von einer Vielzahl von federnden Faserfäden (111) ist, die sich in einer Längsrichtung von dem distalen Ende des Schaftelements erstrecken, das ausgebildet wird, indem die Vielzahl an Faserfäden (111) teilweise durch Adhäsion haftet, so dass Schleim in der Lage ist, aufgrund der Kapillarwirkung von Poren und/oder Schlitzen zwischen den Faserfäden aufgesaugt zu werden, und einen vorderen Endabschnitt (111a) zum Kontaktieren einer Zielstelle hat, und
eine Außenumhüllung (102), in die die Absorptionseinrichtung (105) entlang einer Schaftrichtung des Schaftelementes beweglich ist, so dass die Absorptionseinrichtung in der Lage ist, aus dem vorderen Ende der Außenumhüllung (102) herausgedrückt zu werden, um die Absorptionseinrichtung mit der Zielstelle in Kontakt zu bringen.

2. Ein Probenahme-Werkzeug (101) nach Anspruch 1, wobei die Absorptionseinrichtung (105) den vorderen Endabschnitt (111a) mit einem abgerundeten Spitzenabschnitt zum Kontaktieren des zu absorbierenden Schleims hat.

3. Ein Probenahme-Werkzeug (101) nach Anspruch 1, wobei die Absorptionseinrichtung einen mittleren Abschnitt (111b), der einem Zylinderabschnitt äquivalent ist, und einen hinteren Endabschnitt (111c), der mit dem Schaftelement (106) verbunden ist, hat.

4. Ein Probenahme-Werkzeug (101) nach einem der vorhergehenden Ansprüche, wobei die Faserfäden Außendurchmesser von 0,1 mm oder weniger haben.

5. Ein Probenahme-Werkzeug (101) nach einem der vorhergehenden Ansprüche, wobei die Absorptionseinrichtung (105) eine Porosität von 40% oder mehr hat.

6. Ein Probenahme-Werkzeug (101) nach einem der vorhergehenden Ansprüche, wobei die Absorptionseinrichtung (105) einen Außendurchmesser von 0,5 mm bis 3 mm hat.

7. Ein Probenahme-Werkzeug (101) nach einem der vorhergehenden Ansprüche, wobei das Schaftelement ein für Röntgenstrahlung undurchlässiges Element (131) aufweist, das am distalen Ende des Schaftelements angeordnet ist.

8. Ein Probenahme-Werkzeug (101) nach Anspruch 1, das ferner aufweist:
eine Führungseinrichtung, die die Absorptionseinrichtung (105) über ein Endoskop in einen Körperhohlraum führt, und
eine Drückeinrichtung, die die Absorptionseinrichtung (105) aus der Außenumhüllung (102) an einer Zielverletzung herausdrückt.

9. Ein Probenahme-Werkzeug (101) nach einem der vorhergehenden Ansprüche, wobei die Absorptionseinrichtung (105) 5 bis 60 oder mehr Mikroliter des Mediums im Bezug auf Wasser absorbieren kann.

10. Ein Probenahme-Werkzeug (101) nach einem der vorhergehenden Ansprüche, wobei das Schaftelement (106) ein Draht ist, dessen proximaler Abschnitt abgeknickt ist, um einen abgeknickten Abschnitt (117) zu bilden.

## Revendications

1. Outil de prélèvement (101) comprenant :
- un élément d'arbre (106) ayant une extrémité distale et une extrémité proximale ;
- un absorbeur (105) agencé au moins à l'extrémité distante de l'élément d'arbre (106) et relié à l'élément d'arbre (106),
- ledit absorbeur (105) étant un faisceau d'une pluralité de filaments de fibres résilientes (111) s'étendant dans une direction longitudinale à partir de l'extrémité distale de l'élément d'arbre formé en faisant en partie adhérer la pluralité de filaments de fibres (111) de manière à ce que le mucus puisse être aspiré par capillarité de pores et/ou de fentes entre les filaments de fibres et ayant une partie d'extrémité avant (111a) pour le contact avec la zone cible, et
- une gaine externe (102) à l'intérieur de laquelle ledit absorbeur (105) est déplaçable le long d'une direction d'arbre de l'élément d'arbre de manière à ce que l'absorbeur puisse être pousser vers l'extérieur de la partie d'extrémité avant de la gaine externe (102) pour mettre l'absorbeur (105) en contact avec la zone cible.

2. Outil de prélèvement (101) selon la revendication 1, dans lequel ledit absorbeur (105) a une partie d'extrémité avant (111a) avec une partie du bout arrondie pour entrer en contact avec le mucus destiné à être absorbé.

3. Outil de prélèvement (101) selon la revendication 1, dans lequel ledit absorbeur (105) a une partie centrale (111b) qui est équivalente à un partie cylindrique et une partie d'extrémité arrière (111c) reliée à l'élément d'arbre (106) .

4. Outil de prélèvement (101) selon l'une quelconque des revendications précédentes, dans lequel lesdits filaments de fibres ont un diamètre externe de 0,1 mm ou moins.

5. Outil de prélèvement (101) selon l'une quelconque des revendications précédentes, dans lequel ledit absorbeur (105) a une porosité de 40% ou plus.

6. Outil d'échantillonnage (101) selon l'une quelconque des revendications précédentes, dans lequel ledit absorbeur (105) a un diamètre externe compris entre 0,5 mm et 3 mm.

7. Outil de prélèvement (101) selon l'une quelconque des revendications précédentes, dans lequel ledit élément d'arbre comprend en outre un élément (131) opaque aux rayons X agencé à l'extrémité distale dudit élément d'arbre.

8. Outil de prélèvement (101) selon la revendication 1, comprenant en outre :
- des moyens de guidage de l'absorbeur (105) à l'intérieur d'une cavité du corps à travers un endoscope, et
- des moyens pour pousser l'absorbeur (105) à l'extérieur de la gaine externe (102) au niveau d'une lésion cible.

9. Outil de prélèvement (101) selon l'une quelconque des revendications précédentes, dans lequel ledit absorbeur peut absorber entre 5 et 60 microlitres ou plus en terme de teneur en eau.

10. Outil de prélèvement (101) selon l'une quelconque des revendications précédentes, dans lequel l'élément d'arbre (106) est un fil dont la partie proximale est pliée pour former une partie repliée (117).
